Europäisches Patentamt

European Patent Office

Office européen des brevets

(19)

(11) Publication number: **0 053 869**
**B1**

(12) **EUROPEAN PATENT SPECIFICATION**

(45) Date of publication of patent specification: **01.08.84**

(21) Application number: **81301996.5**

(22) Date of filing: **06.05.81**

(51) Int. Cl.³: **C 12 M 3/02, B 01 F 13/08,**
**B 01 F 7/30, B 01 L 11/00**

(54) **Method and apparatus for stirring particles in suspension such as microcarriers for anchorage-dependent living cells in a liquid culture medium.**

(30) Priority: **04.12.80 GB 8038917**
**12.01.81 EP 81300116**

(43) Date of publication of application:
**16.06.82 Bulletin 82/24**

(45) Publication of the grant of the patent:
**01.08.84 Bulletin 84/31**

(84) Designated Contracting States:
**AT BE CH DE FR GB IT LI LU NL SE**

(56) References cited:
**DE - C - 316 499**
**FR - A - 1 030 658**
**FR - A - 1 600 352**
**FR - A - 2 300 130**
**FR - A - 2 336 476**
**FR - A - 2 447 965**
**GB - A - 353 240**
**GB - A - 2 054 397**
**US - A - 2 958 517**

(73) Proprietor: **TECHNE CAMBRIDGE LIMITED**
**Duxford**
**Cambrigde CB2 4PZ (GB)**

(72) Inventor: **Cooke, George Frederick**
**7 Lime Grove**
**Royston Hertfordshire (GB)**
Inventor: **Pearson, James Moffat**
**Squirrels Leap, Kings Mill Lane**
**Great Shelford Cambridge, CB2 5EN (GB)**

(74) Representative: **Allsop, John Rowland**
**c/o Edward Evans & Co. Chancery House 53-64**
**Chancery Lane**
**London, WC2A 1SD (GB)**

Courier Press, Leamington Spa, England.

## Description

Field of the invention

The present invention relates to a method and apparatus for stirring particles in suspension and particularly for stirring microcarriers or beads within a liquid culture suspension to enable the growth of anchorage-dependent living cells thereon.

Background of the invention

The growth of living cells in a stirred liquid culture medium is well established and is important both in the research and industrial fields.

Some cell types can be grown in a stirred suspension culture without the need for the presence of an attachment surface. However, certain cell types exist which can grow only in the suspension by being termed anchorage-dependent.

In recent years methods for growing anchorage-dependent cells in suspension cultures have been developed using microcarriers, see for example US Patent Specification Serial No. 4,036,693. Thus use of such microcarriers is more economic relative to other known methods as it provides a large surface area for growth in relation to the volume of culture.

Normal methods for cell growth in a liquid culture medium containing microcarriers, involve the necessity to maintain the microcarriers in uniform suspension while avoiding damage to growing anchorage-dependent cells.

One accepted method of stirring cell cultures has been a horizontally revolving permanent magnet either suspended by some form of bearing or as a free component allowed to find its own position in a flat bottomed culture flask. One such device is described in US Patent Specification Serial No. 3,572,651. Such devices are, however, not satisfactory for stirring microcarrier cultures as their minimum threshold speed is high enough to cause damage to the cells and vortices and stagnant zones are created within the stirred medium. In particular a point of stagnation develops immediately below the axis of rotation of the magnet leading to conditions of uncontrolled stirring within the culture medium and consequent nonuniformity of suspension of the microcarriers. Moreover the amount of heat generated by the motor driving the magnet is so great as to render the equipment unsuitable for use in an incubator without employing refrigeration.

A superior type of stirrer for microcarriers suspended in a liquid culture medium which obviates this problem, is disclosed in UK Patent Specification 1,485,741. Such a stirrer is basically comprised of a stirring rod for immersion in a liquid to be stirred contained in a flat bottomed cylindrical culture flask, the stirring rod being provided with means for imparting to it an oscillatory pivotal motion such that the end of the rod describes an orbital path within the culture flask.

One disadvantage of this type of stirrer, as with any stirrer designed to impart a rotational primary movement to a culture liquid within a flat bottomed cylindrical vessel, is that due to the effects of secondary motion within the liquid an accumulation of suspension particles occurs at the outer perimeter and the centre of the bottom of the culture vessel.

The explanation for this accumulation is well known. For example reference may be made to "The cause and formation of meanders in the course of rivers and the so called "Beers Law" from "Ideas & Opinions" by Albert Einstein (Alvin & Redway Ltd, 1954).

In brief, the rotation of the liquid medium during the stirring action, which may be termed the primary motion of circulation, causes a centrifugal force to act on it. At the walls of the stirring vessel the liquid is restrained by friction, so that the angular velocity at which it circulates is less at the walls than in other places near the centre. In particular the angular velocity of circulation of the liquid medium, and hence the centrifugal force will be smaller near the bottom of the vessel than higher up.

The net result will be a vertical circulating movement or secondary circulating motion as opposed to the horizontal or primary circulating motion, of the liquid medium in the vessel. The suspension particles are swept by the secondary motion into the centre and corners of the bottom of the vessel, which effectively form stagnant zones during stirring within the vessel, and accumulate there with time.

Summary of the invention

An object of the invention is to provide a method and apparatus for stirring particles in suspension particularly microcarriers in a liquid culture medium which obviates the risk of accumulation of the microcarriers at the centre or corner of the stirring vessel by secondary motion. In this way damage to the growing cells is avoided because uniform suspension can be achieved at much gentler stirring speeds thus producing improved cell growth and yield in contrast with the prior art.

According to the invention there is provided apparatus for stirring particles in a liquid medium comprising a stirring vessel, an elongate stirrer extending within the vessel to stir its contents, characterised in that the vessel has a substantially centrally located conical portion forming an annular trough or channel therein, said stirrer extending within the vessel such that the end of the stirrer is movable to sweep said trough or channel, which is contoured in section to or substantially to the flow lines at the bottom of the vessel set up by secondary motion stirring effects within the vessel.

The principal advantage of the stirring apparatus according to the invention is that during stirring the dead or stagnant zones at the bottom of the vessel set up by secondary motion effects in the stirred medium are deterred from forming thereby preventing the accumulation of particles in these zones. In this way it becomes possible to stir at much lower speeds than heretofore possible while still maintaining the particles in uniform suspension.

This result is of considerable importance in cell growth research employing stirred microcarriers in liquid culture mediums, because the lower stirring speeds, coupled with better uniformity of suspension of the microcarriers, mitigate the attendant possibility of damage to the growing cells, in all giving much improved cell growth and yield.

Other features and advantages of the present invention will become apparent from the description that follows.

Brief description of the drawings

An embodiment of the invention will now be described by way of example with reference to the accompanying drawings wherein:

Figure 1 is a part cross-sectional view of stirring apparatus for stirring particles in a liquid suspension;

Figures 2A and 2B are illustrative of the two types of circulating motion which are set up in a liquid being stirred in the stirring vessel of the apparatus shown in Figure 1;

Figure 3 is a graphical illustration showing curves of the cell yield rate achieved with apparatus according to an embodiment of the present invention compared with that achieved using the prior art magnetic spinner stirring apparatus;

Figure 4 is a graphical illustration similar to Figure 3 but wherein the culture volume stirred is greater;

Figure 5 is a graphical illustration similar to Figure 3 but wherein the stirring apparatus according to an embodiment of the invention is operated at a minimum stirring speed;

Figure 6 is a graphical illustration similar to Figure 3 but employing a different cell type;

Figure 7 is a cross-sectional view of stirring apparatus according to another embodiment of the invention;

Figure 8 is a detail of the magnetic drive device for operating the stirrer of the apparatus of Figure 7;

Figure 9 is a sectional view through an embodiment of mounting means for mounting the stirrer rod in the stirring vessel of the stirring apparatus of Figures 1 and 7; and

Figure 10 is a sectional view through another embodiment of mounting means for mounting the stirrer rod in the stirring vessel of the stirring apparatus of Figures 1 and 7.

Best modes for carrying out the invention

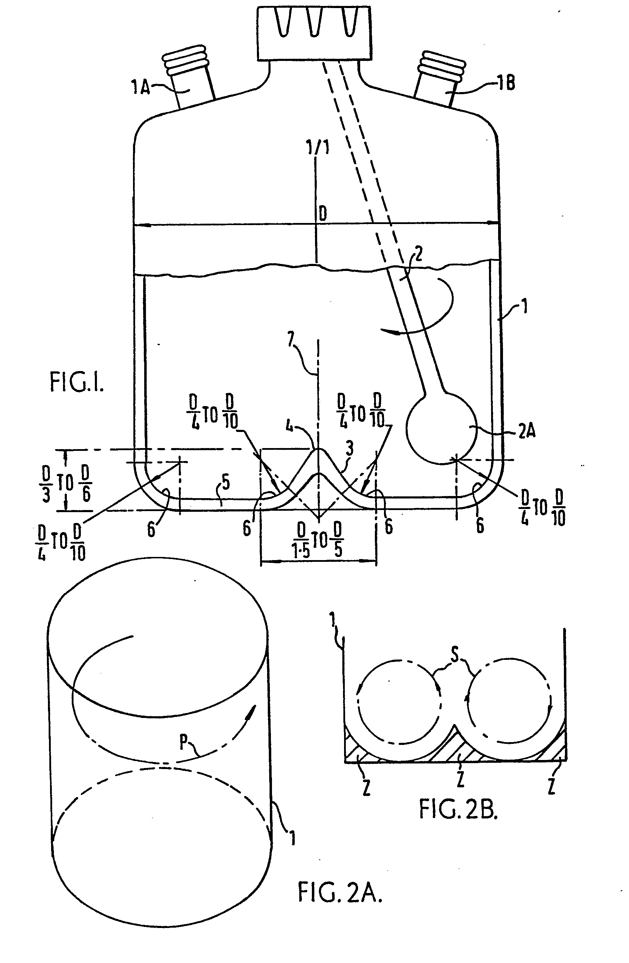

A cylindrical stirring vessel 1 having a diameter D, for use in stirring microcarriers in a liquid culture medium is shown in Figure 1. The vessel 1 is provided with ports 1A and 1B giving access to the culture medium for sampling and control of atmosphere. A stirrer rod 2 preferably made of a moulded polypropylene and having a spherical tip 2A, is shown mounted in the vessel 1 for stirring its contents, the stirrer rod being adapted, by means (not shown) to be moved such that its end describes an orbital path within the vessel, the stirring action closely resembling that of an ordinary hand-held stirring rod.

The bottom of the cylindrical vessel has an upstanding conical portion 3 which tapers to a rounded apex 4 within the interior of the flask 1, to form an interior annular trough or channel 5. The axis of the cone-shaped portion 3 lies on the central vertical axis 7 of the vessel.

The bottom corners of the trough or channel 5 are radiused at 6 as shown. The radiusing however may be continuous in the sense that the trough or channel 5 is formed by being wholly radiused between the inner wall surface of the conical portion 3 interior of the vessel.

The radiused dimensions of the trough or channel 5 are within the range 1/4 to 1/10 the diameter of the vessel 1, with the height to the apex 4 of the cone-shaped portion 3 within the range 1/3 to 1/6 of the diameter D, to give a base diameter of the portion 3 within the range of 1/1.5 to 1/5 of the diameter D of the vessel 1. The diameter of the spherical tip 2A lies in the range 1/2 to 1/20 the diameter D of the vessel 1.

These dimensions have been found to be preferable for preventing the accumulation of microcarriers at the bottom of the stirring vessel 1 by secondary motion effects.

The advantages of stirring microcarriers in a liquid culture medium using a vessel as just described may be illustrated with reference to Figures 2A and 2B.

In operation the end of the stirrer rod 2 is arranged to move around the bottom of the flask as shown with an orbital motion so that its bulbous tip 2A sweeps the annular trough or channel 5, to impart lift to the microcarriers and maintain them in uniform suspension.

The action of the stirrer rod 2 will produce, as has been described earlier, two types of circulatory motion within the vessel, namely a primary motion P producing a horizontal circulatory movement of the liquid suspension as shown in Figure 2A, and a second motion S producing a further vertical circulatory movement of the liquid suspension as shown in Figure 2B.

The secondary motion S produces stagnant zones Z at the centre and at the corners of the bottom of the stirring vessel, whereat microcarriers being stirred are prone to collect.

These stagnant zones are not permitted to form during stirring in the apparatus of Figure 1 due to the shape imparted to the bottom of the stirring vessel 1 as described wherein the contours of the trough 5 in section conform to or substantially conform to the flow lines set up during the secondary motion S thus producing a much more effective overall stirring action for maintaining the microcarriers in uniform suspension.

Thus the microcarriers being swept to the centre and corners of the vessel bottom by the secondary motion, instead of being allowed to settle there, are immediately moved back into the stirred medium by the action of the curved surfaces of the trough or channel 5, to be thereafter maintained in suspension by both the primary and secondary motion effects.

This result means that the speed of revolution of the stirrer rod 2 may be much lower than heretofore found possible to maintain the microcarriers in suspension, and thus damage to the growing cells, which is encountered with stirring at high speeds in a flat bottomed vessel to counter secondary motion effects, avoided.

The stirrer rod 2 is rotated within the culture medium at a rotational speed within the range 30 rpm to 80 rpm. This range has been found to ensure that on the one hand at the lower end of the range, the microcarriers are lifted into suspension, and at the higher end the growing cells are not adversely affected. However, this result has been determined during experiments with microcarriers or beads of a specific type.

Thus it may be expected that, with a stirring vessel of specific dimensions, speeds lower than 30 rpm are possible with differing microcarrier types, the overriding advantage being that notwithstanding these possible lower stirring speeds, the microcarriers once lifted into suspension, will remain there due to the shaping as described of the bottom of the stirring vessel. Additionally it is to be noted that the minimum speed for microcarrier bead suspension reduces as the size of the stirring vessel increases. With this in mind stirring speeds as low as 5 rpm or less would be sufficient to maintain the carriers in suspension with the apparatus according to the invention.

Tests have been carried out to compare the performance of the stirring apparatus according to the present invention with that of the prior art, in particular the traditionally used magnetic spinner vessel referred to earlier. In these tests the stirring apparatus of the invention employed an alternating air current stirrer to operate the stirring rod 2 of the type as disclosed in UK Patent 1,485,741 previously referred to. In these trial summaries therefore the stirring apparatus according to an embodiment of the invention as just described with reference to Figure 1 employing the alternating air current stirrer will be referred to as the ACA stirrer system.

The most critical test in the evaluation of microcarrier stirring equipment is its ability to allow the growth of cells on the microcarriers to high culture densities. The ACA stirrer system was tested in function tests with MRC-5 (human fibroblasts) and Vero (African Green monkey kidney) cells. Of the two types of cell, the MRC-5 cells are the more difficult to grow in all culture systems and were therefore selected for use as a sensitive indicator in most of the trials.

Standard microcarrier techniques were used. Briefly, cells were cultured in Dulbecco's modification of Eagle's medium supplemented with 10A (v/v) foetal calf serum, 10 mM HEPES and non-essential amino acids. Cytodex 1 microcarriers were used at a concentration of 3mg/ml. Cultures were inoculated with cells and stirred immediately in the final culture volume.

The cultures were maintained at 37°C in a water bath and were gassed with 95% air/5% $CO_2$ at the inoculation stage and every time the culture was opened for sampling or medium changes. Apart from these moments all cultures were maintained as sealed units. The medium was changed on day 4 of each growth curve when 50% of the spent medium was replaced by fresh medium. Cultures in the ACA stirrer system were compared with those in magnetic spinner vessels containing comparable culture volumes. The stirring speed of the spinner vessel was 50—60 rpm; such speed has been previously shown to be optimal for this type of vessel. Cell number was determined by the nucleus extrusion method (Sandford et al, 1951).

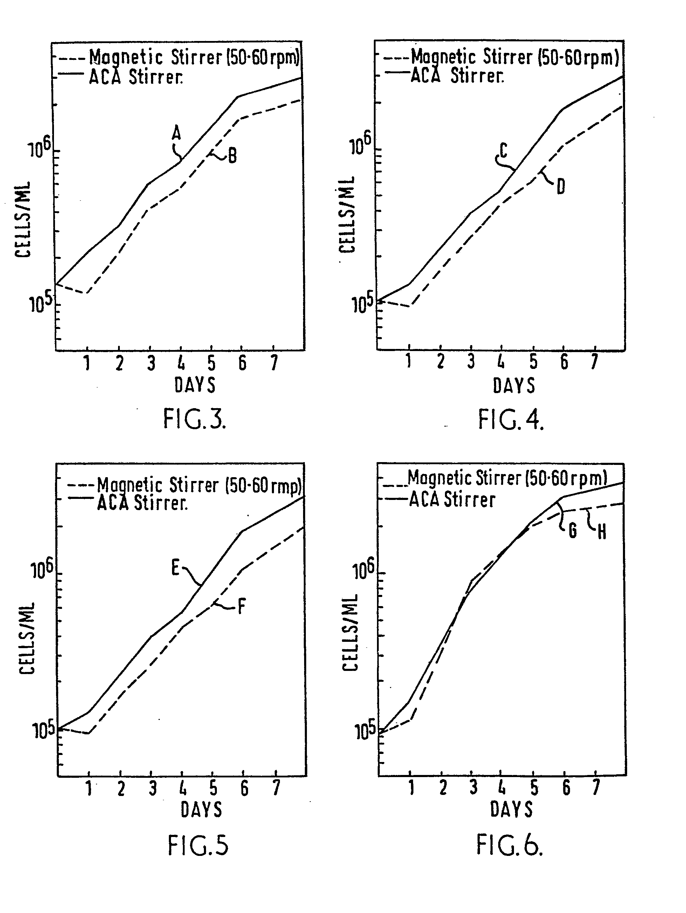

Trial 1 with reference to Figure 3

MCR-5 cells were grown in 450ml microcarrier cultures. In Figure 3 curve A represents the cell yield rate achieved employing the ACA stirrer system while curve B that obtained with the magnetic spinner vessel. The results shown in Figure 3 demonstrates that the ACA stirrer system results in better cell growth and yield than the magnetic spinner vessel. The main effect of the ACA stirrer system was to allow a better plating efficiency of the MRC-5 cells under these culture conditions. The better plating efficiency was reflected by a greater proportion of confluent microcarriers after 8 days and hence a high cell yield. Final cell yield was $2.08 \times 10^6$/ml with the magnetic stirrer and $2.9 \times 10^6$/ml with the ACA stirrer. Thus the ACA stirrer gave a cell yield 40% better than the magnetic stirrer. In both cases the cells had the same maximum population doubling time.

Trial 2 with reference to Figure 4

MCR-5 cells were grown in microcarrier cultures of larger volume, 600ml. This trial confirmed the results of Trial 1 (see Figure 3) in that the ACA stirrer system proved superior to the magnetic spinner vessel, curve C representing the results with the ACA stirrer system and curve D those when employing the magnetic spinner. In this trial, cell yield was $1.87 \times 10^6$/ml in the ACA stirrer. Thus the ACA stirrer system gave a cell yield 57% better than the magnetic stirrer. This increase in cell yield was again due to increase in plating efficiency during the first few hours of culture.

Trial 3 with reference to Figure 5

MRC-5 cells were grown in 450ml microcarrier cultures using a lower stirring speed with the ACA stirrer system. The best result, curve E, was obtained with the slowest possible stirring speed, that is 40 rpm, presumably because of the detachment of mitotic cells from the microcarriers; curve F represents the result obtained with the magnetic stirrer. Slower stirring speeds may result in increased cell yield using the ACA stirrer system but there is the possibility that due to the poorer lift in larger culture volumes slower stirring speeds may result in suboptimal mass gas transfer and therefore supoptimal growth rates.

Trial 4 with reference to Figure 6

In this trial another cell type, Vero, was tested for ability to grow in the ACA stirrer system. This cell type is less sensitive than MRC-5 and has a higher intrinsic plating efficiency. It is probably for this reason that the ACA stirrer system resulted in less improvement over the magnetic spinner vessel, curve G against curve H, than was the case with the MRC-5 trials because Vero cells readily attach to the microcarriers even under the more vigorous conditions prevailing in the magnetic spinner vessel. Nevertheless the final cell yield when using the ACA stirrer system (3.90×10⁶/ml) was 37% better than that obtained with the magnetic spinner vessel (2.87×10⁶/ml).

It will be seen therefore that the ACA stirrer system resulted in cell growth and yield better than that achieved with magnetic spinner vessels. The improved cell growth and yield was most noticeable with the "more hard to grow type" MRC-5.

The apparatus of Figure 1 employing an alternating air current to operate the stirrer rod 2 has proved to be effective for producing much improved cell yield rates compared to that possible with traditional magnetic spinner vessels.

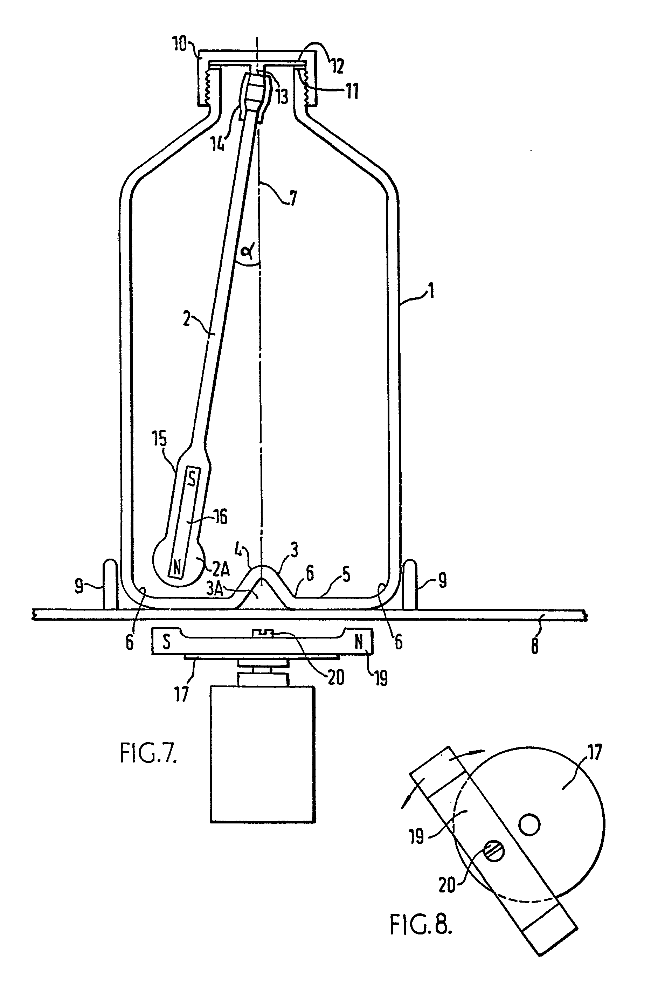

Another method for operating the stirrer rod 2 to produce similar results will now be described with reference to Figure 7.

The stirring apparatus of Figure 7 is similar to that of Figure 1 so that where appropriate like parts when referred to in the following description will be given the same reference numerals.

The vessel 1 in Figure 7 is shown mounted on a plastics surface 8 having three upstanding locating pegs 9, (only two of which are shown) for retaining the vessel 1 in position on the plastics surface 8.

Although not shown the plastics surface 8 could have an additional upstanding locating peg for engaging in the hollow 3A of the conical portion 3.

The neck of the vessel 1 is threaded as shown and is engaged by a correspondingly threaded cap 10 to hermetically seal the vessel 1.

The rim of the neck of the vessel 1 is provided with a sealing washer 11 which is surmounted by a moulding 12.

The moulding 12 has a centrally depending cylindrical projection 13. A piece of silicon rubber tubing 14 is inserted on the projection 13 as shown, the projection 14 being fluted at its end to assist retention of the tubing 14 thereon. One end of the stirrer rod 2 is also inserted in the rubber tubing, this end of the stirrer rod 2 being also fluted to assist retention within the tubing 14. It will thus be appreciated that the silicon rubber tubing 14 constitutes a flexible joint being the projection 13 and the stirrer rod 2.

The tip 15 of the stirrer rod houses a slave magnet 16, the axis of the magnet lying along the axis of the stirrer rod 2. In the embodiment shown the north pole of the magnet 16 is in the lowermost position within the tip 15.

A disc 17 is mounted for rotation on a motor 18 beneath the vessel 1. The motor 18 is positioned such that the axis of the disc lies on the axis 7 of the vessel 1 which passes through the apex of the conical portion 3 and the axis of the projection 13.

A further elongate magnet 19 is mounted by means of a screw 20 on the disc 17 as more clearly shown in Figure 3. The screw 20 passes through the centre of the magnet 19, the position of the screw 20 on the disc 17 being offset with respect to the centre of the disc.

Thus it will be appreciated that the attraction between the south pole of the further magnet 19 and the north pole of the magnet 16 in the rod 2 causes the rod 2 to swing on its flexible bearing 14 such that its axis is at an angle $\alpha$ to the axis 7 of the vessel 1, subsequent rotation of the disc 17 causing the free end of the stirrer rod 2 to sweep out the channel 5 of the vessel in an orbital path, while the stirrer rod 2 remains irrotational around its own axis.

Such an arrangement as described has shown itself to be eminently useful for stirring microcarriers in a liquid culture medium since it does not require connections to be made through the vessel to its interior and thus the necessary hermetic sealing is much easier to achieve. Moreover the elimination of a rotatable bearing for the rod 2 ensures clog-free operation of the apparatus, and the simple magnetic coupling between the revolving magnet 19 and the magnet 16 within the rod 2 obviates the need to employ complicated and consequently expensive, driving means for the rod 2.

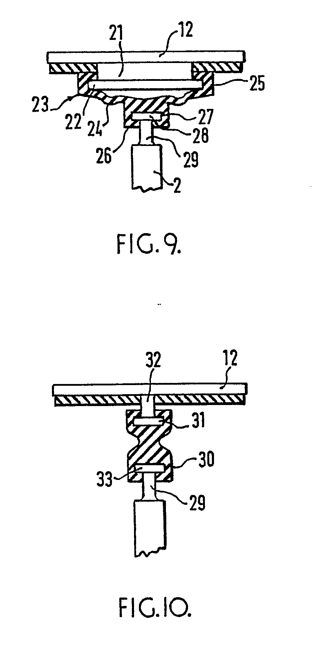

The mounting arrangement for the rod 2 shown in Figures 1 and 7 may be alternatively of the design shown in Figure 9 or Figure 10.

In Figure 9 the mounting member 12 is provided with an integrally formed depending projection 21 bearing an annular rim 22. A moulded flexible connection 23 having a flexible diaphragm 24, is held to the rim 22 by a cooperating annular recessed head portion 25 of the connector 23.

The diaphragm 24 is provided with a downwardly projecting integrally formed flexible support piece 26 having a recess 27 shaped to receive in gripping engagement the head 28 of a T-shaped terminating extension piece 29 of the rod 2.

The rod support embodiment of Figure 10 employs a solid waisted flexible connection piece 30 between the rod 2 and the mounting member 12 as distinct from the connecting piece comprising the tubing 14 as shown in Figure 7.

Each end of the waisted connecting piece 30 is provided with a T-shaped recess 31 housing in gripping engagement on the one hand a correspondingly configured T-shaped projecting member 32 of the mounting member 12, and on the other hand the T-shaped extension piece 29 of the rod 2 as shown in Figure 9.

Both the embodiments of Figure 9 and 10 form a more positive attachment of the rod 2 to its flexible mounting within the stirring vessel compared to that provided by the tubular connecting piece 14 of the stirring apparatus shown in Figure 7. In all cases however the arrangements shown provide a flexible mounting for the stirrer rod with no rotating bearings enabling the free end of the rod to be rotated in an orbital path while remaining irrotational around its own axis.

The stirring apparatus as described herein, when used in cell culture research, is capable of producing improved cell yield rates over the prior art, particularly the traditional magnetic spinner vessels and stirring systems employing orbiting stirrer rods in flat bottomed culture vessels.

It has additionally been shown that greater culture yields are possible with the apparatus of the present invention when intermittent short periods of agitation are introduced during the attachment phase of culture growth. To provide such intermittent stirring action, a timer may be connected to the power line of the motor 18 shown in Figure 7, causing the motor 18 to switch on for short intervals of time, typically 30 seconds on and 300 seconds off.

Furthermore, to minimise transient disturbances to the cultures, the stirrer rod may be caused to accelerate slowly to steady running speeds, and to decelerate slowly to stop. This is achieved by incorporating a suitable time constant, typically 5 seconds, in the power circuit of the motor 18, to cause the voltage applied to the motor to increase or decrease slowly when switched on or off respectively.

The invention will now be defined in the following claims but it will be appreciated to those skilled in the art that modifications to the apparatus as described are possible which would still fall within the scope of some or all of these claims. For example although the invention has been exemplified with reference to a cylindrical vessel, nevertheless vessels of other shapes and cross-section would equally be suitable such as a conical stirring vessel.

**Claims**

1. Apparatus for stirring particles in a liquid medium comprising a stirring vessel (1), an elongate stirrer (2) extending within the vessel (1) to stir its contents, characterised in that the vessel (1) has a substantially centrally located conical portion (3) forming an annular trough or channel (5) therein, said stirrer (2) extending within the vessel such that the end of the stirrer is movable to sweep said trough or channel, which is contoured in section (6) to or substantially to the flow lines at the bottom of the vessel (1) set up by secondary motion stirring effects (S) within the vessel (1).

2. Apparatus for stirring the contents of a vessel as claimed in Claim 1 characterised by means (10, 12, 13, 14, 23, 30) for supporting the stirrer (2), within the vessel (1), said supporting means (10, 12, 13, 14, 23, 30) being capable of allowing said end to move in said orbital path while the stirrer (2) remains irrotational around its own axis.

3. Apparatus as claimed in Claim 2 wherein said supporting means comprises a mounting member (12) which sits on the rim of an open neck of said vessel (1), said mounting member (12) having a projection (13, 21, 32) depending internally of the vessel (1), a flexible link (14, 23, 30) between the projection (13, 12, 32) and the other end of said stirrer (2), and a closure cap (10) for said open neck retaining said mounting member (12) in position on said rim.

4. Apparatus as claimed in Claim 3 characterised in that a first magnet (16) is positioned in said end of the stirrer (2), a second magnet (19) positioned exteriorly of the vessel (1) to attract the first magnet (16) and cause the axis of the stirrer to lie at an angle to the axis (7) of the vessel (1), and means (19, 18) for rotating the second magnet such that under the mutual attraction between the first and second magnets said end of the stirrer (2) rotates in said orbital path.

5. Apparatus as claimed in Claim 4 characterised in that said second magnet (19) is mounted on a disc (17) by means of a screw (20), the screw (20) being positioned on the disc (17) offset with respect to the centre of the disc, whereby to allow rotatable adjustment of said second magnet (19) on the disc and thus the diameter of orbit of said end of the stirrer (2).

6. Apparatus as claimed in Claim 5 characterised in that the central axis of the vessel (7) passes through the axis of said depending projection (14) and the centre of the disc (19).

7. Apparatus as claimed in Claim 6 characterised in that the vessel is cylindrical, said trough or channel (5) being radiused, at the least, at its corners respectively between the inner wall of the vessel (1) and between the interior surface of the conical portion (3) and the bottom of the vessel (1), and at the most, the whole extent of its interior surface between the inner wall of the vessel (1) and the apex (4) of said conical portion (3).

8. Apparatus as claimed in Claim 7 characterised in that the radiused dimensions of the trough or channel (5) are within the range 1/4 to 1/10 the diameter (D) of the vessel (1), the height of the conical portion (3) being within the range 1/3 to 1/6 the diameter (D) of the vessel (1).

9. Apparatus as claimed in Claim 8 characterised in that the base diameter of said conical portion (3) is within the range 1/1.5 to 1/5 the diameter (D) of the vessel (1).

10. Apparatus as claimed in Claim 9 characterised in that said elongate stirrer (2) has a bulbous tip (2A) at said end thereof which sweeps said annular trough or channel (5), the dimensions of said bulbous tip (2A) being within the range 1/2 to 1/20 the diameter (D) of the vessel (1).

11. A method of stirring particles in a liquid medium characterised by stirring the particles in a stirring vessel having a substantially centrally located conical portion forming an annular trough or channel in the bottom of the vessel, the trough or channel being contoured to or substantially to the flow lines set up in the vessel by the effects of secondary stirring motion, the end of the stirrer being moved within the vessel in an orbital path to sweep out said annular trough or channel to maintain the particles in uniform suspension.

12. A method as claimed in Claim 11 characterised in that the end of the stirrer is moved in said orbital path irrotationally around its own axis.

13. A method as claimed in Claim 12 characterised in that the stirrer is moved within the vessel at a speed within the range 5 rpm to 80 rpm.

14. A method as claimed in any one of Claims 11 to 13 characterised in that the particles are microcarriers being stirred in a liquid culture medium.

15. A method as claimed in Claim 14 characterised in that the microcarriers are stirred intermittently.

16. A method as claimed in Claim 12 characterised in that the stirrer is accelerated slowly from rest to achieve a desired steady state stirring speed, and then decelerated slowly to zero after a predetermined time of stirring at said steady state speed.

**Revendications**

1. Appareil pour agiter des particules dans un milieu liquide comportant un vase d'agitation (1), un agitateur de forme allongée (2) s'étendant à l'intérieur du vase (1) pour remuer son contenu, caractérisé en ce que le vase (1) présente une partie (3) de forme conique substantiellement disposée au centre et déterminant dans le vase un creux ou canal annulaire (5), le dit agitateur (2) s'étendant à l'intérieur de vase de telle manière que son extrémité puisse balayer le dit creux ou canal (5) dont le bord est relevé en coupe (6) de façon à suivre substantiellement au fond du vase (1) les lignes de flux établies par des effets (S) du mouvement d'agitation secondaire à l'intérieur du vase (1).

2. Appareil pour agiter le contenu d'un vase selon la revendication 1, caractérisé en ce que des moyens de maintien (10, 12, 13, 14, 23, 30) de l'agitateur (2) sont prévus à l'intérieur de vase (1), les dits moyens de maintien (10, 12, 13, 14, 23, 30) permettant à la dite extrémité de se éplacer selon une course orbitale tandis que l'agitateur (2) reste fixé en rotation autour de son propre axe.

3. Appareil selon la revendication 2, caractérisé en ce que les dits moyens de maintien comprennent un élément de support (12) reposant sur le bord de l'ouverture du col du dit vase (1), le dit élément de support (12) présentant une partie (13, 21, 32) faisant saillie à l'intérieur du vase (1), un joint flexible (14, 23, 30) reliant cette partie (13, 21, 32) et l'autre extrémité du dit agitateur (2), et un bouchon (10) fermant l'ouverture du dit col et maintenant en position le dit élément de support (12) sur le dit bord.

4. Appareil selon la revendication 3, caractérisé en ce qu'il comprend en outre un premier aimant (16) disposé à la dite extrémité de l'agitateur (2), un second aimant (19) disposé à l'extérieur de vase (1) de manière à attirer le premier aimant (16) et obligeant l'axe de l'agitateur à présenter un angle par rapport à l'axe (7) du vase (1), et des moyens (19, 18) pour mettre le second aimant en rotation de telle manière qu'il résulte de l'attraction mutuelle entre les premier et second aimants le déplacement de la dite extrémité de l'agitateur (2) selon la dite course orbitale.

5. Appareil selon la revendication 4, caractérisé en ce que le dit second aimant (19) est fixé sur un disque (17) au moyen d'une vis (20), cette dernière étant décalée par rapport au centre du disque (17), de manière à permettre l'ajustement en rotation du dit second aimant (19) sur le disque et, par suite, le réglage du diamètre de l'orbite de la dite extrémité de l'agitateur (2).

6. Appareil selon la revendication 5, caratérisé en ce que l'axe central (7) du vase est aligné avec l'axe de la partie faisant saillie (13) et le centre du disque (19).

7. Appareil selon la revendication 6, caractérisé en ce que le vase affecte une forme cylindrique, le dit creux ou canal (5) étant circulaire au moins à ses angles respectifs entre la paroi interne du vase (1) et entre la surface intérieure de la partie conique (3) et le fond du vase (1) et, au plus, sur toute la surface intérieure entre la paroi interne du vase (1) et le sommet (4) de la dite partie conique (3).

8. Appareil selon la revendication 7, caractérisé en ce que les dimensions radiales du creux ou canal (5) sont comprises entre 1/4 et 1/10 du diamètre (D) du vase (1), la hauteur de la partie conique (3) étant comprise entre 1/3 et 1/6 du diamètre (D) du vase (1).

9. Appareil selon la revendication 8, caractérisé en ce que le diamètre de base de la dite partie conique (3) est compris entre 2/3 et 1/5 du diamètre (D) du vase (1).

10. Appareil selon la revendication 9, caractérisé en ce que la dite extrémité l'agitateur de forme allongée (2) qui balaye le dit creux ou canal (5) présente une forme de bulbe (2A) dont les dimensions sont comprises entre 1/2 et 1/20 du diamètre (D) du vase (1).

11. Procédé pour agiter des particules dans un milieu liquide caractérisé en ce que l'on agite les particules dans un vase d'agitation présentant une partie de forme conique substantiellement disposé au centre et déterminant un creux ou canal annulaire au fond du vase, les bords du creux ou canal étant relevés de façon à suivre substantiellement les lignes de flux établies, dans le vase pour les effets du mouvement d'agitation secondaire, l'extrémité de l'agitateur étant déplacé dans le vase selon une course orbitale de manière à balayer le dit creux ou canal annulaire pour maintenir les particules en suspension uniforme.

12. Procédé selon la revendication 11, caractérisé en ce que l'extrémité de l'agitateur suit une course orbitale sans tourner autour de son propre axe.

13. Procédé selon la revendication 12, caractérisé en ce que la vitesse de l'agitateur dans le vase est comprise entre 5 et 80 tours par minute.

14. Procédé selon l'une quenconque des revendications 11 à 13, caractérisé en ce que les particules sont des micro-supports qui sont agités dans un milieu de culture liquide.

15. Procédé selon la revendication 14, caractérisé en ce que les micro-supports sont agités de façon périodique.

16. Procédé selon la revendication 12, caractérisé en ce que l'on accélère lentement l'agitateur à partir de sa position de repos jusqu'a ce qu'il atteigne une vitesse stable souhaitée d'agitation, puis est décéléré lentement jusqu'à une vitesse nulle après un temps déterminé d'agitation à la dite vitesse stable.

## Patentansprüche

1. Vorrichtung zum Umrühren von Partikeln in einem flüssigen Medium, mit einem Rührgefaß (1) und einen langgestreckten Rührelement (2), welches sich innerhalb des Gefäßes (1) erstreckt, un den Inhalt des Gefäßes umzurühren, dadurch gekennzeichnet, daß das Gefäß (1) einen im wesentlichen mittig angeordneten konischen Abschnitt (3) aufweist, der eine ringförmige Rinne oder einen rinnen-förmigen Kanal im Gefäß bildet, daß sich das Rührelement (2) innerhalb des Gefäßes in der Weise erstreckt, daß das Ende des Ruhrelementes bewegbar ist, um sich entlang der Rinne oder des Kanals zu bewegen, die bzw. der im Schnitt eine Randlinie (6) augweist, die den durch einen Sekundär-Rühreffekt (S) Innerhalb des Gefäßes (1) bedingten Flußlinien am Boden des Gefäßes (1) angepaßt bzw. im wesentlichen angepaßt ist.

2. Vorrichtung zum Umrühren des Inhaltes eines Gefäßes nach Anspruch 1, gekennzeichnet durch Mittel (10, 12, 13, 14 , 23, 30) zur Halterung des Rührelementes (2) innerhalb des Gefäßes (1), wobei die Mittel zur Halterung (10, 12, 13, 14, 23, 30) eine Bewegung des erwähnten Endes auf der erwähnten Kreisbahn ermöglichen, während das Rührelement (2) um' seine eigene Achse rotationsfrei bleibt.

3. Vorrichtung nach Anspruch 2, dadurch gekennzeichnet, daß die Mittel zum Halten ein Halteelement (12), welches am Rand eines offenen Halses des Gefäßes (1) sitzt und einen in das Innere des Gefäßes (1) reichenden Vorsprung (13, 21, 32) besitzt, eine flexible Verbindung (14, 23, 30) zwischen dem Vorsprung (13, 12, 32) und dem anderen Ende des Rührelementes (2) sowie eine Verschlußkappe (10) für den offenen Hals aufweist, un das Halteelement (12) in seiner Position an dem Rand zu halten.

4. Vorrichtung nach Anspruch 3, dadurch gekennzeichnet, daß ein erster Magent (16) in dem erwähnten Ende des Rührelementes (2) angeordnet ist, daß ein zweiter Magnet (19) außerhalb des Gefäßes (1) angeordnet ist, un den ersten Magnet anzuziehen und um zu bewirken, daß die Achse des Rührelementes mit der Achse (7) des Gefäßes (1) einen Winkel einschließt, und daß Mittel (19, 18) vorgesehen sind, un dem zweiten Magneten rotierend in der Weise anzutrieben, daß aufgrund gegenseitigen Anziehung zwischen dem ersten und zweiten Magneten das Rührelement auf der erwähnten Kreisbahn rotiert.

5. Vorrichtung nach Anspruch 4, dadurch gekennzeichnet, daß der zweite Magnet mit Hilfe einer Schraube (20) an einer Scheibe (17) montiert ist, daß die Schraube (20) exzentrisch zur Mitte der Scheibe an dieser Scheibe (17) angeordnet ist, un durch Drehen eine Einstellung des zweiten Magneten (19) an der Scheibe und dadurch eine Einstellung des Durchmessers der Kreisbahn des erwähnten Endes des Ruhrelementes (2) zu ermöglichen.

6. Vorrichtung nach Ansprung 5, dadurch gekennzeichnet, daß die Mittelachse des Gefäßes (7) achsgleich mit der Achse des nach unten reichenden Vorsprungs (14) sowie achsgleich mit der Mittelachse der Scheibe (19) verläuft.

7. Vorrichtung nach Anspruch 6, dadurch gekennzeichnet, daß das Gefäß zylinderförmig ausgebildet ist, daß die Rinne bzw. der Kanal (5) zumindest an den Ecken zwischen der Innenwand des Gafäßes (1) bzw. zwischen der Innenfläche des konischen Abschnittes (3) und dem Boden des Gefäßes (1) und vor allem auch der gesamte Bereich der Innerfläche zwischen der Innerwand des Gefäßes (1) und der Spitze (4) des konischen Abschnittes (3) gekrümmt sind.

8. Vorrichtung nach Anspruch 7, dadurch gekennzeichnet, daß die Krümmungsradien der Rinne oder des Kanales (5) innerhalb eines Bereiches von 1/4 bis 1/10 des Durchmessers (D) des Gefäßes liegen, und daß die Höhe des konischen Abschnittes (3) innerhalb eines Bereiches von 1/3 bis 1/6 des Durchmessers (D) des Gefäßes (1) liegt.

9. Vorrichtung nach Anspruch 1, dadurch gekennzeichnet, daß der Basisdurchmesser des konischen Abschnittes (3) innerhalb eines Bereiches von 1/1.5 bis 1/5 des Durchmessers (D) des Gefäßes (1) liegt.

10. Vorrichtung nach Anspruch 9, dadurch gekennzeichnet, daß das langgestreckte Rührelement (2) eine knollenartige Spitze (2A) an dem erwähnten einen Ende aufweist, welches sich entlang der ringförmigen Rinne bzw. des ringförmigen Kanals (5) bewegt, und daß die Abmessungen der erwähnten knollenartigen Spitze (2A) im Bereich zwischen 1/2 bis 1/20 des Durchmessers (D) des Gefäßes (1) liegen.

11. Verfahren zum Umrühren von Partikeln in einem flüssigen Medium, gekennzeichnet durch Unrühren der Partikel in einem Rührgefäß, welches einen im wesentlichen mittig angeordneten konischen Abschnitt aufweist, der eine ringförmige Rinne oder einen ringförmigen Kanal im Boden des Gefäßes bildet, wobei die Rinne oder der Kanal den Flußlinien, die im Gefäß durch die Wirkungen einer Sekundär-Unrühr-Bewegung herrühren, angepaßt oder im wesentlichen angepaßt sind, und wobei das Ende des Rührelementes im Gefäß auf einer Kreisbahn bewegt wird, um die erwähnte ringförmige Rinne oder den erwähnten ringförmigen Kanal zu bestreichen und um dadurch die Partikel in einer gleichmäßigen Verteilung zu halten.

12. Verfahren nach Anspruch 11, dadurch gekennzeichnet, daß das Rührelement auf seiner Kreisbahn ohne Rotation um die eigene Achse bewegt wird.

13. Verfahren nach Anspruch 12, dadurch gekennzeichnet, daß das Rührelement innerhalb des Gefäßes mit einer Geschwindigkeit im Bereich zwischen fünf Umdrehungen pro Minute bis achtzig Underhungen pro Minute bewegt wird.

14. Verfahren nach einem der Ansprüche 11—13, dadurch gekennzeichnet, daß die Partikel Mikroträger sind, die in einem flüssigen Kultur- oder Nähr-Medium umgerührt werden.

15. Verfahren nach Anspruch 14, dadurch gekennzeichnet, daß die Mikroträger intermittierund umgerührt werden.

16. Verfahren nach Anspruch 12, dadurch gekennzeichnet, daß das Rührelement aus der Ruhestellung langsam auf eine gewünschte konstante Rührgesschwindigkeit beschleunigt wird und dann langsam auf Null abgebremst wird, und zwar nach einer vorgegebenen Rührzeit bei der erwähnten konstanten Rührgeschwindgkeit.

FIG.I.

$\frac{D}{4}$ TO $\frac{D}{10}$

$\frac{D}{3}$ TO $\frac{D}{6}$

$\frac{D}{4}$ TO $\frac{D}{10}$

$\frac{D}{4}$ TO $\frac{D}{10}$

$\frac{D}{1.5}$ TO $\frac{D}{5}$

$\frac{D}{4}$ TO $\frac{D}{10}$

FIG.2A.

FIG.2B.

FIG.3.

FIG.4.

FIG.5

FIG.6.

FIG.7.

FIG.8.

FIG. 9.

FIG. 10.